# NEW EUROPEAN PATENT SPECIFICATION

(11) **EP 1 322 960 B2**
(45) Date of publication and mention of the opposition decision: **13.02.2013**
(45) Mention of the grant of the patent: 05.01.2005
(21) Application number: 01980473.1
(22) Date of filing: 03.10.2001
(51) Int. Cl.: G01N 33/68

(54) **ALLERGEN-MICROARRAY ASSAY**
ALLERGEN-ASSAY AUF BASIS VON MIKROANORDNUNGEN
TEST DE JEU ORDONNE DE MICROECHANTILLONS D'ALLERGENES

(30) Priority: 03.10.2000 EP 00890296
(43) Date of publication of application: 02.07.2003
(73) Proprietor: Phadia Multiplexing Diagnostics GmbH, 1030 Wien (AT)
(72) Inventor: HILLER, Reinhard, A-1120 Vienna (AT); HARWANEGG, Christian, A-1100 Vienna (AT); MÜLLER, Manfred, W., A-3400 Klosterneuburg (AT)
(74) Representative: HOFFMANN EITLE
(86) International application number: PCT/EP2001/011429
(87) International publication number: WO 2002/029415

(56) References cited:
- EP-A- 0 556 745
- US-A- 3 720 760
- US-A- 4 444 879
- US-A- 4 849 337
- MENDOZA L G ET AL: "High-throughput microarray-based enzyme-linked immunosorbent assay (ELISA)." BIOTECHNIQUES, vol. 27, no. 4, October 1999 (1999-10), pages 778-788, XP000992893 ISSN: 0736-6205 cited in the application

## Description

The present invention relates to a method for the detection of an IgE immunoglobulin which binds to an allergen in a sample as well as a method for in vitro diagnosis of allergies in a patient.

An allergy is a reaction produced by the body's immune system to a substance that would normally be thought of as harmless. It is this response that causes the symptoms that are classed as allergic reactions. Allergy is therefore not a failure of the immune system, but its over activity. The response of an allergic person to an allergen can produce a wide range of symptoms. Some people suffer symptoms such as asthma, eczema, rashes, itchy eyes, sinusitis, blocked or runny nose and hay fever, however, more serious symptoms can occur. With allergies such as those to venoms, nuts and shellfish, for example, a potentially life threatening condition called anaphylactic shock can occur. This happens when the body produces a reaction so severe that the throat swells, blood pressure drops and the person has difficulty in breathing. In some cases this type of reaction can be fatal.

The incidence of allergies is increasing in the developed countries (i.e. in Europe, Northern America and Japan)..Estimates range from 20-50% of the population being affected. It is now known that allergies are the result of an unbalance in the T-cell compartment of the immune-system. More precisely, allergies are accompanied by an increased activity of so-called T-helper 2 (Th2) cells relative to T-helper 1 (Th1) cells, giving rise to increased IgE production.

IgE (immunoglobulin E) is at least one of the substances that cause allergic reactions. IgE specific for an allergen is not normally detected in the blood and is only produced when a person becomes sensitised to a substance. Substances that cause an allergy (called an allergen) produce a specific IgE that is unique to and will only react with it. This reaction between IgE and the allergen is like a lock and key. IgE, when combined with the allergen, causes cells to release chemicals (e.g. histamine), which cause the symptoms of allergy. A person may have specific IgE to more than one substance and may therefore be allergic to more than one substance. Results for IgE testing are expressed as a grade that indicates how much IgE specific to the substance tested for, is present in the blood. The higher the grade the more likely the patient is to be allergic. Over the past decades allergic diseases have been diagnosed using only scarcely purified extracts derived from the major allergen sources. Evidently, these crude mixtures are complex and variable in composition. As a consequence, the diagnostic potential of these extracts may vary and lead to the production of false-negative results. The latter has been ascribed mainly to the instability of allergens in extracts. Another drawback of using extracts in the diagnosis of allergies is the poor standardisation of commercially available products with respect to their allergenic composition. Units that express the potencies and methods of calculation are different for each company in the market. As a consequence, diagnostic products from different companies can hardly be compared.

The most important disease-related components, the major allergens, have been identified over the past decades, but their quantification is not used as the basis for standardisation of allergen extracts. Monoclonal antibodies against most major allergens are now available.

Allergy testing according to known methods is not a straightforward process. These methods can be useful procedures provided that a history is taken to identify which tests would be most appropriate. However, there are only very few recognised medical tests that can identify allergies and as a general rule these have been restricted to clinical laboratories or specialist centres. Whilst the diagnosis of atopy or allergy by a qualified practitioner or specialist in allergy can be relatively easily made further tests are often required to confirm this.

There are various types of allergy testing:
1. Skin Prick testing
   Suspected allergens are injected just under the surface of the skin and the reaction is observed by a qualified nurse or doctor. As the reaction develops there is a zone of redness, the stronger the reaction the greater the zone. Skin testing is quite sensitive although not all allergies can be identified by this method.
2. Patch testing
   Patch testing may be useful in cases of contact dermatitis. Test substances are usually applied to the skin covered by a patch and left in place for 48 hours. A positive reaction produces a small area of eczema. Again the reaction is observed by a qualified nurse or doctor.
3. Alternative allergy testing
   There are many alternative types of allergy testing and unfortunately few are accurate or recognised by the medical profession. These alternative tests are often misleading as well as costly.
4. Vega test
   The Vega test is an electrical test described as bioenergetic regulatory technique. The machine measures conductivity with a Wheatstone circuit. Electrodes are connected to acupuncture points or held in the patient's hand. Different solutions are then placed in a metallic tray. The machine is then calibrated by placing a glass vial containing a toxic substance into the tray. The vial causes a reduction in electrical conductivity. Other substances are then placed into the tray and if they give a similar reading this is reported as an allergic or "sensitive" reaction. This technique is widely used in health food stores however, its value is unproved and there are no valid trials that will substantiate claims.
5. Leucocytotoxic test
   The Leucocytotoxic involves mixing the patient's white blood cells with an extract of specific food and then measuring the cells in different ways for evidence of some form of change. There are a high number of false positive and false negative reactions and the American Academy of Allergy concluded that there was no evidence that the test was effective for the diagnosis of food or inhalant allergy.
6. Hair analysis
   Another form of testing is hair analysis. The hair can be analysed for the presence of toxic metals such as Lead, Mercury and Cadmium or low levels of Selenium,' Zinc, Chromium, Manganese and Magnesium. Heavy metal poisoning is well recognised and documented in forensic science and hair analysis can indicate exposure to metals. However, hair analysis as a means to diagnosing allergy, through methods such as "dowsing", have never been validated.
7. Applied kinesiology
   Samples of food are placed under the tongue or held in a glass container in the hand of the patient. The patient is then asked to push his free arm against that of the examiner. If an allergic response is detected this manifests as a reduced muscle response and the patient experiences difficulty in raising his arm. This technique fails to with stand up against a double blind study.
   Furthermore, there are various in vitro methods for diagnosing allergies which detect the presence of IgE or IgG antibodies in the blood of a patient:
8. Conventional methods as ELISA or Western blots are used to detect antibodies (IgE) present in serum of a patient with the help of (recombinant) allergens.
9. Radioallergosorbent test (RAST®):
   In this procedure a specific allergen is coupled to a paper disk; immunospecific IgE, if present in the test (patient's) serum will bind to the disk; detection is effected by radiolabeled anti-IgE. Different scoring systems comparing test results with the absolute binding of a negative control are in use. Commonly, the modified RAST® procedure is followed with overnight incubation and results in higher sensitivity.
10. RAST based quantitative IgE inhibition experiments in which allergen extract is dotted on nitro-cellulose strips. Aliquots of sera are preincubated with a mixture of specific recombinant allergens after which this mixture is incubated on the nitro-cellulose strips. Bound IgE is detected with anti human IgE antibodies. The percentage inhibition of IgE binding the natural extracts after preabsorption with recombinant allergens is calculated in a last step.
11. Cellular antigens stimulation test (CAST) according to which patient basophilic cells are stimulated with interleukin-3 and allergen followed by measurement in an ELISA of de novo generated and released sulfidoleukotriene (SLT).
12. UniCAP: Recombinant allergens are immobilized on a solid phase structure after which the binding of antibodies which are present in the serum of a patient to the allergens is detected.

However, these methods require a multitude of individual experimental steps in order to test a larger number (e.g. 100) different allergens. Furthermore, these above mentioned tests do not show a sufficiently high sensitivity and reliability and are very time consuming. Also the amounts of sample (e.g. serum of a patient) as well as purified, possibly recombinant, costly allergens which are needed to carry out these tests are large.

The US 4 444 879 relates to methods and apparatus for immnoassassays to determine total immunoglobulin and IgE. Here allergens are extracted and immobilized in polymer coated wells of a microtiterplate. The sample to be analyzed is then added to the well and a conventional immuno assay is carried out.

In the EP 0 556 745 A1 a method for the detection of anti-wheat-protein IgE antibodies in body fluids is described whereby a wheat allergen extract is immobilized onto a solid phase which is for example chromatographic or capillary material or fibre, glass, nylon, cellulose or derivatives thereof in form of glass beads, microparticles, sheets or wells of a microtiterplate. Also here a conventional immunoassay is carried out in order to detect antibodies in a sample of a patient.

The US 3 720 760 relates to a method for analyzing body fluid for immunoglobulins. Also here allergen containing extracts which are commercially available are attached to water insoluble polymer after which a conventional immuno assays is carried out.

The US 4 849 337 relates also to a method for identifying allergen specific IgE levels in a patient serum by conjugating the IgE in the serum with allergens adhering to an insoluble support, Here again the allergen may be an extract or an allergen derived directly from polens, dusts, animals, etc.

The detection of allergen-specific immunoglobulins by ELISA with extracts of allergens bound to a solid surface is disclosed in US 4,444,879 A and EP 0 556 745 A. Mendoza et al. (Biotechniques 27(4) (1999)) describe a high-throughput microarray-based ELISA.

These conventional immuno assay methods, however, are not useful to test a patient with respect to the presence of allergies since the amount of different allergies has mounted to over a few hundred and is increasing steadily. The time and work necessary to analyze a patient's serum for all possible end rare allergies is too much and will not be carried out in laboratories where a few hundred patient's samples must be analyzed daily.

The object of the present invention is therefore to provide a method for the detection of an IgE immunoglobulin which binds to an allergen in a sample which does not show the above mentioned drawbacks and which can be carried out in a short time with only a small amount of sample and allergens, which is highly reliable and sensitive and most importantly allows the detection of a practically unlimited number of allergens in one single assay.

A further object of the present invention is to provide a method for in vitro diagnosis of allergies in a patient without the above mentioned drawbacks.

The above mentioned method is characterized in that one or more purified single allergens are immobilized on a microarray chip after which the sample is incubated with the immobilized allergens so that immunoglobulins which are specific for the allergens bind to the specific allergen after which the IgE immunoglobulins which are bound to the specific immobilized allergens are detected.

Microarrays have been adopted recently for a number of DNA manipulating techniques that are established in the scientific community since long. These DNA chips have become available in a number of different formats and will eventually change ways of designing experiments in the ordinary laboratory work. In vitro DNA diagnosis has become less time-consuming and labor-spending since this novel technology allows assay complexity in a high-throughput format. Consequently, in the area of proteome research, classic solid phase substrates, such as microtiter plates, membrane filters and microscopic slides are being turned into the high-density microarray format. The new and versatile protein array technology eventually allows high-throughput screening for gene expression and molecular interactions (Walter et al., Curr Opin Microbiol 2000 Jun;3(3):298-302; Emili & Cagney, Nat Biotechnol 2000 Apr;18(4)393-7). Recently, the concept of protein arrays has been adopted for its use in immunological assays.

A biochip is described as capable of supporting high-throughput (HT), multiplexed enzyme-linked immunosorbent assays (ELI-SAs). These biochips may consist for example of an optically flat glass plate containing 96 wells formed by an enclosing hydrophobic Teflon mask, however, also other materials and forms of biochips are known and used. Experiments demonstrate that specific multiplex detection of protein antigens arrayed on a glass substrate is feasible. Further application of this new high-throughput screening (HTS) format include direct cellular protein expression profiling, multiplexed assays for detection of infectious agents and cancer diagnostics (Mendoza LG et al, Biotechniques 1999 Oct;27(4):778-80).

However, in these known microarray chip methods testing proteins the antibodies are immobilized to the microarray chip. Surprisingly, it has been found that it is possible not only to bind the antibodies to the microarray chip but even allergens which are the antigens corresponding to specific antibodies, in particular IgE and IgG, respectively. This fact is particularly surprising since functional allergens show a particular secondary structure which may be modified when bound in such high density to a solid phase as the microarray chip. This modification of the structure of the allergen would interfere with the antibody-allergen binding which would lead to false negative results. Antibodies, fragments of antibodies or peptides are relatively small and show high stability, therefore antibodies show a greater stability in solution relative to their cognate antigens. However when immobilized to a solid support even in the case of antibodies only a small percentage remain intact and active.

The article by Haab et al. (Genom Biology 2000, I (6): pre-print 0001,1-0001,22) which was received on 9 November 2000 relates to protein microarrays for detection and quantitation of proteins and antibodies in solutions. The results of the analysis carried out according to this publication show that only 50% of the arrayed antigenes and 20% of the arrayed antibodies provided specific and accurate.measurements of the cognate allergens.

Moreover, diversity of possible allergens is of course much higher than antibody diversities with respect to handling the proteins, especially for immobilizing and staining such a series of structurally diverse allergens. However, it has been surprisingly shown that allergens immobilized on a microarray chip show high reliability and sensitivity in a method for the detection of IgE immunoglobulins in a sample.

This method allows to carry out an assay for a multitude of IgE immunoglobulins which bind to a specific allergen in one experimental step which assay can also be atomised: More than 100 different allergens can be tested without having to carry out a multitude of individual experimental steps as when performed in a conventional microtiter form. Furthermore, this assay shows a high degree of sensitivity and reliability. Also, the test results of this method can be obtained within a short period of time, e.g. about three hours, shortening the assay time when compared to conventional RAST or ELISA tests.

Furthermore, the microarray assay is highly reproducible when performing repetitive experiments with chips containing samples from different persons, e.g. by a microtiter plate-like mask whereby every well comprises a number of spots of different allergens and a sample of one person is added per well. A further advantage of this method according to the present invention is due to the fact that a large number of different probes occupies a relatively small area providing a high density. The small surface area of the array permits extremely uniform binding conditions (temperature regulation, salt content, etc.) while the extremely large number of probes allows massively parallel processing of hybridizations. Because the high density arrays contain such a large number of probes it is possible to provide numerous controls including, for example, controls for variations or mutations in a particular allergen, controls for overall hybridization conditions, controls for sample preparation conditions, and mismatch controls for non-specific binding or cross hybridization.

Further, the assay requires only a minimal fraction of material, (allergen as well as a sample) when compared to conventional test systems. This means that with an equal amount of starting material a much greater number of test kits can be manufactured when using the microarray form and a smaller amount of sample can be used to test for a larger amount of IgE immunoglobulins which bind to allergens. Also, in small volumes, binding may proceed very rapidly.

Natural intact "immunoglobulins" or antibodies comprise a generally Y-shaped tetrameric molecule having an antigen binding-site at the end of each upper arm. An antigen binding site consists of the variable domain of a heavy chain associated with the variable domain of a light chain. More specifically, the antigen binding site of an antibody is essentially formed by the 3 CDRs (complementarity determining regions) of the variable domain of a heavy chain (V.sub.H) and the 3 CDRs of the variable domain of the light chain (V.sub.L).

Generally the term "antigen" refers to a substance capable of eliciting an immune response and ordinarily this is also the substance used for detection of the corresponding antibodies by one of the many in vitro and in vivo immunological procedures available for the demonstration of antigen-antibody interactions.

Similarly, the term "allergen" is used to denote an antigen having the capacity to induce and combine with specific (i.e., IgE) antibodies which are responsible for common allergies; however, this latter definition does not exclude the possibility that allergens may also induce reactive antibodies, which may include immunoglobulins of classes other than IgE.

"Immobilizing" in the context of the proteins or peptides refers to the binding or attaching of the protein/peptide to solid supports by conventional means, with or without an additional spacer between the solid support and the allergen. Immobilizing peptides/proteins to a support is well known in the art; in the scope of the present invention any immobilization is comprised, e.g. covalent, non-covalent, in particular by hydrophobic interactions with for example membranes and synthetic surfaces, respectively, etc.

A "microarray" is an array of features (e.g. "spots") having a density of discrete features of at least about 16/cm², preferably at least about 64/cm², still preferred about 96/cm² and most preferred at least about 1000/cm². The features in a microarray have typical dimensions, e.g., diameters, in the range of between about 10-2000 µm, preferably about 50-500 µm, still preferred about 150-250 µm, and are separated from other features in the array by about the same distance. Therefore, a microarray according to the present invention useable for routine mass screening may have at least 500, preferably at least 1000 individual spots comprising allergens, standards and controls.

The "chip" according to the present invention may be of virtually any shape, e.g. a slide or a well of a microtiter plate, or even a multitude of surfaces although a planar array surface is preferred.

The detection step can be carried out with any conventional method known by the person skilled in the art e.g. by physical, enzymatic, chemical reaction, etc.

According to the present invention IgEs are detected as immunoglobulins. IgE is known as a substance that causes allergic reactions and is only produced when a person becomes sensitive to a substance, an allergy. Therefore, in order to test if the sample is from a patient who is allergic to the specific allergen the sample is tested for IgE as immunoglobulins. The higher the amount of IgE in the sample the more likely the patient the sample is taken from is allergic to the specific allergen.

IgG is known to be present in a sample of a patient who is allergic to food.

Advantageously one or more purified allergens are immobilized on the microarray chip. These - single - allergens are for example purified from an allergen extract, e.g. from a specific food or plants. Of course, one or more allergens of a specific species can be analysed at once.

The methods of purification are known to the person skilled in the art, e.g. chromatographic purification, purification by mass separation, purification by specifically binding the allergen to a solid phase, e.g. over an antibody, etc. Application of highly purified allergens for mass production of allergy testing has not yet been proposed.

"Purified" allergens relate - to the contrary of extracts - to single allergens which can be for example native, recombinant or synthetically produced allergens. These purified allergens show a number of important advantages over allergen extract when immobilized to a solid support: Due to the mixture of various proteins which are present in any extract the concentration of allergens to be tested is very low compared to purified single allergen preparations. Purified single allergens can therefore be immobilized to the microarray in a very high concentration. Due to the exact and defined molecules present in a preparation comprising purified allergens only defined allergens will be immobilized to the micro array. Therefore, the microarray and the method for detecting antibodies with the help of purified allergens can be standardized and subjected to precise quality controls.

Furthermore, due to the high concentration of purified allergens in the composition the allergens are immobilized at a higher density on the microarray and therefore any detection method with the help of such a microarray is more sensitive than corresponding microarrays with extracts comprising allergens. Another advantage of purified single allergens over allergen extracts lies in the fact that the purified allergens immobilized are exactly defined. To the contrary of purified allergens allergen extracts comprise for example two, three or more different allergens of one organism or object. For example, in the case of an apple, an extract will comprise a mixture a different apple allergens, whereas the inventive composition will comprise one of the purified apple allergens.

A further advantage of purified allergens with respect to non-purified allergens for example present in extract lies in the fact that due to the additional impurities present in the extract the extract is not stable for more than a certain amount of time and can for example go mouldy. This will, however, influence the detection of allergies since also allergies against mould exists in which case a false positive result would occur.

Furthermore, due to the presence of proteases in the extract the extracts are instable which is not the case with purified allergens.

Also, due to the at present existing therapies with the help of purified allergens a diagnosis with the same purified allergen components would be a major advantage over diagnosis with unpurified allergens. Such diagnoses are particularly advantageous in order to follow the therapy with purified allergens and to test the individual reaction to a specific therapy and thereby to provide a "patient tailored" therapy.

For the above reasons a method for the detection of an IgE immunoglobulin which binds to the allergen in a sample whereby one or more purified allergens are immobilized to the microarray chip is particularly advantageous.

Preferably one or more recombinant allergens are immobilized on the microarray chip. Over the last few years a number of recombinant allergens have been produced. The production of recombinant allergens is also in principle known state of the art but had little impact on routine allergy testing. By using recombinant allergens it is possible to provide a great number of one or more specific allergens and therefore to optimize sensitivity of the test. It is furthermore possible to modify the recombinant allergens specifically in order to produce any allergen mutations to detect for specific IgE immunoglobulins. Furthermore, making use of the major allergens as recombinant proteins provides an alternative to the extract based tests with respect to assay stability as well as to diagnostic standardisation. The above mentioned advantages of purified allergens over the unpurified (extract) allergens apply even more for recombinant allergens than for purified native allergens: Recombinant allergens can be even more specifically designed than the purified native allergens and therefore it is possible to optimize affinity and specifity of a test with recombinant allergens. Recombinant allergens are also better to standardize with respect to their production method. Moreover, differences between production lots are smaller for recombinant allergens than for allergens derived from natural sources. It has been surprisingly shown with the present invention that the use of these recombinant allergens in the routine in vitro diagnostic tests according to the present invention is superior to conventional extract based test systems. In contrast to the routine serial testing according to the state of the art, the present allergen testing using also recombinant allergens brings a completely new quality with respect to standardisation, controllability, sensitivity, reproducibility, the ability to test for diagnostic relevant information, etc. for standard allergen testing.

Recombinant allergens are for example those described in the publications by Chapman et al. Allergy, 52:374-379 (1997), Laffer et al. J Allergy Clin Immunol Vol 98 Number 2 652-658 (1996), Müller et al. Clinical and Experimental Allergy Vol 27 9. 915-920 (1997), Niederberger et al. J Allergy Clin Immunol Vol 102 Number 4, Part 1 579-591 (1998), Menz et al. Clinical and Experimental Allergy Vol 26, 50-60 (1996) which are incorporated herein by reference. Further examples of (recombinant) allergens are but not limited to rBetv1, rBetv2, rBetv4, rJunO2, Cass1, rPhlp1, rPhlp2, rPhlp4, rPhlp5a, rPhlp6, rPhlp7, rPhlp11, rPhlp12, rParj2, Artv1a, Mugwort profilin, Apig1, Apig1.0201, Dauc1.2, rArah2, rArah5, Maldl, Mald2, rPena1, recCarp, rDerp1, rDerp2.0101, rDerp2, rDerp2b, rDerp5, rDerp5a, rDerp7, rDerp8, rDerp10, rTyrp2, rLepd2.01, rLepd13, rEurm2.0101, rFeld1, rFeld1a, rBosd2, a representative allergen from Cat, a representative allergen from Dog, a representative allergen from BSA, a representative allergen from Mouse, a representative allergen from Rat, a representative allergen from Pig, a representative allergen from Sheep, a representative allergen from Chicken, a representative allergen from Rabbit, a representative allergen from Hamster, a representative allergen from Horse, a representative allergen from Pigeon, a representative allergen from Guineaalbumin, HSA, a-NAC, rPenc3, Penn13, rPenc19a, rPenc19b, rAspf1, rAspf1a, rAspf3, rAspf3a, rAspf4, rAspf4a, rAspf6, rAspf6a, rAspf7, rAspf8, rAlta1, rAlta2, rMalf1, rMalf5, rMalf6, rMalf7, rMalf8, rMalf9, rHevb1, rHevb1a, rHevb3, rHevb8, rHevb9, rHevb10, rHevb11, rAK, rBlag2, rBlag4, rBlag5, Phospholipase.A, Hyaluronidase, rVesv5, rVesg5.

According to a further advantageous embodiment one or more synthetically produced allergens are immobilized on the microarray chip. Here again the same advantages over allergen extract and also over purified native allergens apply as mentioned above for recombinant allergens. The method for synthetically producing peptides or proteins is well known in the state of the art; by synthetically producing allergens it is possible to provide highly purified allergens in a great number and at low costs since such production methods are highly automatized. Furthermore, the exact sequence of any allergen can be provided with or without modifications, which increases the sensitivity and reliability of the method for detecting the IgE immunoglobulins.

It is also possible to use only the allergenic determinant or the allergenic domain of a specific allergen in the test according to the present invention. This may eliminate the risks and drawbacks of working with large proteins, because shorter peptidic structures are easier to handle for routine production of biochips. This applies to all purified native, recombinant and synthetic allergens. This would further allow to detect single peptide epitopes which would still improve the diagnostic test and therapies in particular with respect to specificity.

It is of particular advantage to provide the native form of the allergen as well as a synthetic or recombinant form of the allergen on the same chip. Whereas it is preferred to use mainly recombinant or synthetic allergens the native form of an allergen may be provided at least as a control or additional quality information on the chip. A preferred chip according to the present invention therefore comprises a native and a synthetic or recombinant form of at least one allergen. This provides also for improved quality control and standardisation of different batches of allergens.

Preferably one or more haptens are immobilized as allergens on the microarray chip. A "hapten" is a low molecular weight (typically weighing less than about 7000 Daltons) substance that is generally incapable of causing, by itself, a significant production of antibodies upon administration to.an animal body, including a human body. This can occur because a hapten is too small to be recognized by the body's immune system. However, when a hapten is coupled to a larger, carrier molecule, the hapten can acquire antigenic properties. In other words, binding the hapten to the carrier molecule (to make an analyte-carrier molecule combination) permits the bound hapten to be recognized by an animal's immune system. An immunoprecipitation reaction can take place between the hapten (coupled to the carrier molecule), and an antibody to the hapten. By providing haptens which are immobilized to the microarray chip a higher density can be achieved on the chip.

It is of course possible to combine these above mentioned different forms of allergens, e.g. to use at the same time recombinant and (purified) native allergens. The combination of these different forms allows to compare them and carry out a control of the e.g. recombinant allergens but also as a control for the various native lots of the allergens which may vary due to differences in biological sources, methods of preparation, purity, etc.

For a highly efficient test it is preferable to use allergens which show optimal features, e.g. a high binding capacity. However, the use of less or differently active antigens is preferred for the detection of inefficiently binding allergen-variants, e.g. for the use in hyposensitization therapies.

According to a preferred embodiment of the present invention the allergens are immobilized on a 10 to 2000 µm diameter, preferably 50-500 µm diameter, still preferred 150-250 µm diameter, spot on the microarray chip. Since the spots have a small diameter, it is possible to immobilize a great amount of different allergens and various concentrations of specific allergens on separate spots of the microarray chip, thus allowing to provide one microarray chip which can in one - automatized - step be used to analyse a big number of allergens or allergen concentrations.

Preferably the allergens are immobilized on a solid support, preferably a glass carrier, synthetic carrier, silicon wafer and membrane, respectively. Such chips can be produced for example according to methods described in Ge, H. (2000), Nucleic Acids Research, 28, e3 (i-vii); Qian W. et al. (2000), Clinical Chemistry, 46 (1456-1463); MacBeath G. et al. (2000), Science, 289, (1760-1763), which are disclosed herein by reference. Each of these materials shows specific characteristics and advantages which are well-known to the person skilled in the art. Therefore, the material for the microarray chip will be chosen according to the allergen which is to be tested, the method for the.detection of bound IgE immunoglobulins, the used buffers. Furthermore, the choice of material is also a financial question.

Preferably the microarray chip is chemically modified, preferably by aminoreactive and carboxyreactive modification, respectively. This allows to precisely determine the way the allergen is bound to the microarray chip.

Advantageously the allergens are covalently bound to the microarray chip. This provides a stable binding of the allergens to the microarray chip, thereby providing a method which is particularly reliable.

According to another advantageous embodiment of the present invention the IgE immunoglobulins are detected in blood serum as the sample. In patients who are allergic to an allergen immunoglobulins are produced mainly in the blood serum, so that analyzing the blood serum provides a reliable method for detecting IgE immunoglobulins. Furthermore, blood serum can easily be collected from the patient in a very simple way and the collection of the sample can be carried out even at the home of the patient.

Preferably the blood serum is diluted 1:1 - 1:15, preferably 1:5. The dilution can be carried out with any suitable solution, e.g. 1x TBST (10 mM Tris-HCl, pH 8,0, 150 mM NaCl, 0,5% TWEEN 20)

Advantageously the sample is incubated 1 min. to 24 hours, preferably 1 - 2 hours, with the allergens. Of course, it is possible to incubate the sample with the allergens even over a period of days. However, this does not result in an increase in signal intensity or specificity. In general, an incubation time of 1 hour is sufficient for a reliable and sensitive result.

Furthermore, the sample is preferably incubated at a temperature between 0 and 60°C, preferably at 37°C, with the allergens. Incubations at lower temperatures do not result in an increase of signal, but they rather lead to an increase in unspecific binding and background noise. An incubation at 37°C has shown to provide exact and reliable results for allergen test in humans.

Advantageously the bound IgE immunoglobulin is detected with at least one labelled specific anti-IGE immunoglobulin antibody. As used herein, the term "antibody" refers to intact molecules as well as fragments thereof, such as Fa, F(ab).sub.2, and Fv, which are capable of binding the epitopic determinant. Antibodies can be prepared using intact polypeptides or fragments containing small peptides of interest as the immunising antigen. The polypeptide or peptide used to immunise an animal can be derived from the transition of RNA or synthesized chemically, and can be conjugated to a carrier protein, if desired. Commonly used carriers that are chemically coupled to peptides include bovine serum albumin and thyroglobulin. The coupled peptide is then used to immunise the animal (e.g., a mouse, a rat, or a rabbit).

In the scope of the present invention the term antibody refers to monoclonal or polyclonal antibodies whereby monoclonal antibodies may be prepared using any technique which provides for the production of antibody molecules by continuos celllines in culture. These include but are not limited to the hybridoma technique, the human B-cell hybridoma technique and the IBV hybridoma technique.

Furthermore, chimeric antibodies may be produced, single chain antibodies as well as synthetically produced antibodies.

The antibody may be labelled according to any known method which allows to qualify and preferably quantify the bound antibody. Detectable labels suitable for use in the present invention include any composition detectable by spectroscopic, photochemical, biochemical, immunochemical, electrical, optical or chemical means. Useful labels include biotin for staining with labelled streptavidin conjugate, magnetic beads, fluorescent dyes (e.g. fluorescein, texas red, rhodamine, green fluorescent protein, and the like), radiolabels (e.g. ³H, ¹²⁵I, ³⁵S, ¹⁴C or ³²P), enzymes (e.g. horse radish peroxidase, alkaline phosphatase and others commonly used in ELISA), and colorimetric labels such as colloidal gold or coloured glass or plastic (e.g. polystyrene, polypropylene latex, etc.) beads.

Means of detecting such labels are well known to those of skill in the art. Thus, for example, radiolabels may be detected using photographic film or scintillation counters, fluorescent labels may be detected using a photodetector to detect emitted light. Enzymatic labels are typically detected by providing the enzyme with a substrate and detecting the reaction product produced by the action of the enzyme on the substrate, and colorimetric labels are detected by simply visualising the coloured label.

Preferably the bound IgE immunoglobulins are detected with at least one fluorescence and radioactive, respectively, labelled specific anti-IgE immunoglobulin antibody. These are classic methods for qualitatively and quantitatively analyzing bound IgE antibody which are very specific and reliable.

Other advantageous detection systems for micro arrays are for example those described in Schult K. et al. (1999), Anal Chem, 71 (5430-5435); Vo-Dinh T. et al. (1999), Anal Chem, 71 (358-363); Brignac SJ Jr. et al. (1999), IEEE Eng Med Biol Mag, 18 (120-122); Otamiri M. et al. (1999), Int J Biol Macromol, 26 (263-268); Wright, GL Jr. et al. (2000), Prostate Cancer and Prostatic Diseases, 2 (264-276); Nelson RW. et al. (2000), Electrophoresis 21 (1155-1163); Rich RL. et al. (2000), Curr Opin Biotechnol 11 (54-61); Chen JJ. et al. (1998), Genomics, 51 (313-324), which publications are enclosed herein by reference.

Advantageously one or more indoor allergens are immobilized as allergens. These may be but are not limited to Mites, Tyr. put, Lep. dest. or .mayrei, Felis, Bos, Albumine, Pen. cit., Pen. not., Asp. fumigatus, Alt. alt., Malassezia furfur, Latex, Plodia, Blatella.

According to a further preferred embodiment one or more outdoor allergens are immobilized as allergens. These may be but are not limited to Betula, Juniperus, Phleum, Parietaria judicea.

Further one ore more food allergens may be immobilized as allergens. Examples are sellerie, karott, peanut, apple, shrimp, fish.

Further one ore more venom allergies are immobilized as allergens. These may be but are not limited to Bee or Wasp.

A further aspect of the present invention relates to a method for in vitro diagnosis of allergies in a patient, whereby a serum sample from the patient is analysed for IgE immunoglobulins which bind to allergens according to an above mentioned method according to the present invention, whereby a microarray chip is used on which at least 10, preferably at least 50, still preferred at least 90, different allergens are immobilized, after which a positive reaction between the sample and the immobilized allergens is diagnosed as an allergy. The above mentioned definitions and advantages relate also to this method for in vitro diagnosis of allergies compared to the known methods for diagnosing allergies. The present method shows the advantage that a big amount of allergies can be simultaneously diagnosed with only one sample since all the allergens which are to be tested are immobilized on one single microarray chip. Every step is therefore carried out only on one chip, whereby the chip further may comprise spots with no immobilized allergens and therefore allowing simultaneous negative detection. The amount of allergens to be tested is not limited and it is of course possible to provide two or more microarray chips.

A further aspect of the present invention relates to the use of a microarray chip on which one or more purified allergens are immobilized for the detection of IgE immunoglobulin. Also in this aspect the above mentioned definitions and advantages are applied.

Preferably the allergens are immobilized on a 100 to 500 µm diameter, preferably to 200-300 µm diameter, spot.

Still preferred the microarray chip is a glass carrier, synthetic carrier, silicon wafer and a membrane, respectively.

Advantageously the microarray chip is chemically modified, preferably by aminoreactive and carboxyreactive modification, respectively.

Advantageously the allergens are covalently bound to the microarray chip.

A further aspect according to the present invention relates to the use of a kit which comprises a microarray chip according to the present invention as mentioned above and a first reagent comprising at least one immunoglobulin detecting reagent, preferably an anti-immunoglobulin antibody, preferably in a known concentration, and possibly a second reagent as a positive sample comprising at least one immunoglobulin which binds to an allergen. The first reagent comprising the at least one immunoglobulin detecting reagent, preferably an anti-immunoglobulin antibody may be used for the detection of bound immunoglobulin in which case the anti-immunoglobulin antibody is preferably labelled as mentioned above. Preferably an antibody is present in the first reagent in a known concentration thereby allowing to provide for reproducible results. Furthermore, the kit preferably comprises a second reagent as a positive sample comprising at least one IgE immunoglobulin which binds to an allergen. Also here it is preferable that the IgE immunoglobulin is present in a known concentration in the second reagent thereby allowing to quantify the IgE immunoglobulin present in the sample by comparing the results of the sample of the patient with the results of positive sample (the second reagent).

The kit is provided for carrying out a method according to the present invention as mentioned above. For this the first and second reagents may be designed in order to detect one specific IgE immunoglobulin which binds to a specific allergen or one specific allergy in a patient. However, the kit may also be designed to detect a variety of IgE immunoglobulins which bind to specific allergens or to diagnose a variety of allergies in a patient.

The present invention is now described in more detail with the following examples and figures to which it is of course not limited.
Fig. 1 shows a layout of an allergen microarray;
Fig. 2 shows a scan image of an allergen array assayed with serum from an allergic person;
Fig. 3a-3c show a Scatterplot Test from a triple assay;
Fig. 4a-4c show the % fluorescence measured for immobilised extract and immobilised recombinant allergens.

### Example 1:

### Allergen spotting

Allergens were arrayed using a GMS 417 spotter from Genetic Microsystems. The proteins were spotted on derivatized glass slides. Individual allergens were spotted at one hit per dot as triplicates. The dots (features) showed a diameter of about 200µm.

The allergens were assembled in functional groups as follows:
(A) Outdoor (I. Trees [1=rBetv1, 2=rBetv2, 3=rBetv4, 4=rJunO2, 5=Cass1], II. Grasses [6=rPhlp2, 7=rPhlp4, 8=rPhlp5a, 9=rPhlp1, 10=rPhlp6, 11=rPhlp7, 12=rPhlp11, 13=rPhlp12], III. Weeds [14=rParj2, 15=Artv1a, 16=Mugwort profilin]),
(C) Food Allergens (X. Vegetables [17=Apig1, 18=Apig1.0201, 19=Dauc1.2, 20=rArah2, 21=rArah5], XI. Fruits [22=Mald1, 23=Mald2], XII. Shrimps [24=rPena1], XIII. Fish [25=recCarp]),
(B) Indoor (IV. Mites [26=rDerp1, 27=rDerp2.0101, 28=rDerp2, 29=rDerp2b, 30=rDerp5, 31=rDerp5a, 32=rDerp7, 33=rDerp8, 34=rDerp10, 35=rTyrp2], V. Animals [36=rLepd2.01, 37=rLepd13, 38=rEurm2.0101, 39=rFeld1, 40=rFeld1a, 41=rBosd2, 42=a representative allergen from cat, 43=a representative allergen from dog, 44=BSA, 45=a representative allergen from mouse, 46=a representative allergen from rat, 47=a representative allergen from pig, 48=a representative allergen from sheep, 49=a representative allergen from chicken, 50=a representative allergen from rabbit, 51=a representative allergen from hamster, 52=a representative allergen from horse, 53=a representative allergen from pigeon, 54=guineaalbumin], VI. Moulds [55=rPenc3, 56=rPenc19a, 57=rPenc19b, 58=Penn13, 59=rAspf1, 60=rAspf3, 61=rAspf4, 62=rAspf6, 63=rAspf1a, 64=rAspf3a, 65=rAspf4a, 66=rAspf6a, 67=rAspf7, 68=rAspf8, 69=rAlta1, 70=rAlta2], VII. Yeast [71=rMalf7, 72=rMalf1, 73=rMalf5, 74=rMalf6, 75=rMalf8, 76=rMalf9], VIII. Latex [77=rHevb1, 78=rHevbla, 79=rHevb3, 80=rHevb8, 81=rHevb9, 82=rHevb10, 83=rHevb11], IX. Insects [84=rAK, 85=rBlag2, 86=rBlag4, 87=rBlag5]),
(D) Venoms (XIV. Bee [88=Ag5, 89=Phospholipase A, 90=Hyaluronidase, 91=rVesv5, 92=rVesg5], XV. Wasp [93=Ag5]),
(E) Auto-allergens (94=HSA, 95=a-NAC)

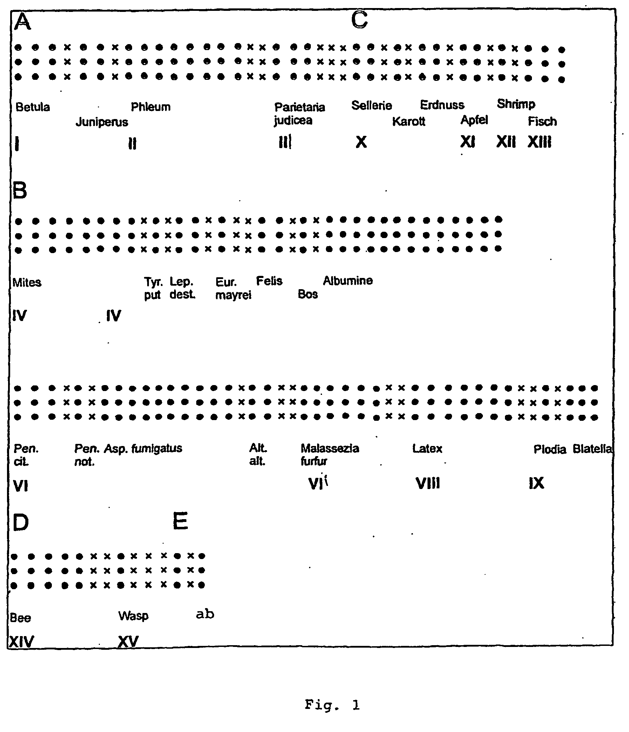

Additionally, buffer dots were interspersed between the allergen subgroups serving as a background control, marked as "X", "ab" is the labelled antibody. (Fig. 1: 1864x1182 pixels, 1,86x1,18 cm, 1000 pixels per cm, pixel depth/colours 8/256, 1 pixel corresponding to 10µm).

100 µl aliquots of the allergens were divided into the wells of a 96 well microtiter plate at a concentration of ∼200 ng/µl in spotting buffer (300 mM Sodium-phosphate pH 8.5). The optimal concentration for immobilization of the allergens was calculated from titration experiments with several allergens in different buffer solutions as well as on different slides. The proteins assayed displayed a saturation behaviour at concentrations equal to or larger than 100 ng/µl as became evident from a constantly strong white signal when scanning with a GMS scanner. At this concentration the binding behaviour of the allergens was independent of the composition of the storage as well as the spotting buffer.

### Example 2:

### SOPHIA (Solid phase Immunosorbent Assay)

Following over night incubation slides either purchased from CEL Associates (aldehyde slides) or prepared in-house were washed in 1x TBST (10 mM Tris pH 8.0/150 mM NaCl/0.5% Tween 20) under vigorous shaking in a Falcon tube at ambient temperature. As a blocking step, the slides were transferred to a 1x TBST solution containing 0.01% BSA for 2 hours at ambient temperature. Successively, slides were washed in 1x TBST for 15 min. and rinsed in distilled water briefly.

The allergen array was incubated with diluted serum (1:5 in 1x TBST) for (at least) 60 min. at 37°C with shaking. Various degrees of serum dilution have been tested, ranging from 1:1 - 1:15. Usually a dilution of 1:5 gave the best results. 30 µl of diluted serum was added to the slides in Press Seal Chambers purchased from SIGMA Technologies. The chambers were used as according to the manufacturers protocol. Various incubation times for the serum ranging from 60 min. to over night were assayed. However, an extended incubation with serum did not result in an increase in signal intensity or specificity. Following the incubation with serum slides were washed in 1x TBST (15 min., ambient temperature) with shaking.

Five different serums of allergic patients that have been examined using conventional diagnostic tests (skin prick test, RAST, ELISA) and a serum of a non-atopic patient were chosen adequate for a benchmark test of the allergen array. The individual sera were assayed at least twice on different batch produced allergen arrays.

Fluorescence-labelled α IgE antibody labelled with AlexaFluor fluorescence dye according to the manufacturer's protocol (Molecular Probes) was added to the allergen array in a solution containing 0.01% BSA/1x TBST and incubated for 60 min. at ambient temperature. Working dilutions for the antibody ranged from 1:1000 - 1:5000 depending on the time and efficiency of labelling. Following the immunoassay, slides were washed with 1x TBST (15 min., ambient temperature, shaking) and briefly rinsed with distilled water.

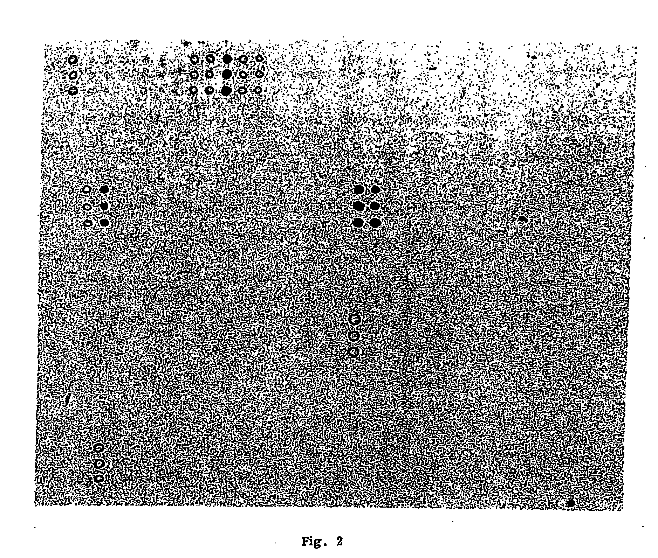

Following the immunosorbent assay, the slides were evaluated with a GMS two-colour scanner. Generally, signal intensities varied only slightly between different assays and different slides. However, the background values differed depending on the type of slides used. An example of a scanned image of an allergen array assayed with serum of a patient suffering of allergy is depicted in Fig. 2. This patient shows a strong reaction with Phleum, Mite, Felis and bee (cf. Fig. 1). No background signals stemming from an unspecific interaction with buffer dots or auto-allergens were observed. Following the complete evaluation of the allergic patient's sera the results were compared with data preliminary obtained for the identical sera using PAST tests. The data achieved with the method according to the present invention were in good agreement with those data sets available

### Example 3:

### Reproducibility test assaying serum of patient C.

Serum of patient C was assayed three times on individually prepared allergen-microarrays. The experimental procedure was as essentially described before.

Following the assay, the slides were scanned using identical hardware settings. Data analysis was performed using the GenePix software package. The calculated mean values of the signal intensities for each allergen triplicate were compared after three repeats of the experiment.

Only values corresponding to signals that were at least 1.5 x higher than the mean value of the buffer spot signals were chosen for the final analysis. The mean value, standard deviation and percentage of standard deviation for the relevant signals are depicted in table 1.

**table 1**

| **Allergen** | **Test 1** | **Test 2** | **Test 3** | **Mean Value** | **Standard Dev.** | **% Mean** |
|---|---|---|---|---|---|---|
| **rBet v 1** | 20731 | 21325 | 25060 | 22372,00 | 1916,11 | **8,56** |
| **rPhl p 2** | 17176 | 15529 | 14227 | 15644,00 | 1206,67 | **7,71** |
| **rPhl p 4** | 36134 | 33511 | 29328 | 32991,00 | 2802,76 | **8,50** |
| **rPhl p 5a** | 56600 | 53068 | 55594 | 55087,33 | 1485,77 | **2,70** |
| **rPhl p 6** | 56244 | 51359 | 37625 | 48409,33 | 7882,14 | **16,28** |
| **rPhl p 12** | 6291 | 7003 | 12289 | 8527,67 | 2675,50 | **31,37** |
| **rPar j2** | 7552 | 7678 | 9444 | 8224,67 | 863,73 | **10,50** |
| **Art v 1a** | 6997 | 7931 | 11258 | 8728,67 | 1828,70 | **20,95** |
| **Mugwort profilin** | 7901 | 7669 | 9260 | 8276,67 | 701,74 | **8,48** |
| **Mal d 1** | 9890 | 15295 | 8176 | 11120,33 | 3033,74 | **27,28** |
| **HSA** | 9868 | 8732 | 10708 | 9769,33 | 809,71 | **8,29** |
| **Sheepalbumin** | 9858 | 8295 | 10222 | 9458,33 | 835,92 | **8,84** |
| **Horsealbumin** | 9602 | 9061 | 10559 | 9740,67 | 619,37 | **6,36** |
| **rAsp f 1(b)** | 12216 | 10129 | 10781 | 11042,00 | 871,77 | **7,90** |
| **rMal f 5** | 13459 | 11018 | 12035 | 12170,67 | 1001,14 | **8,23** |
| **rAK** | 20680 | 14202 | 19978 | 18286,67 | 2902,48 | **15,87** |

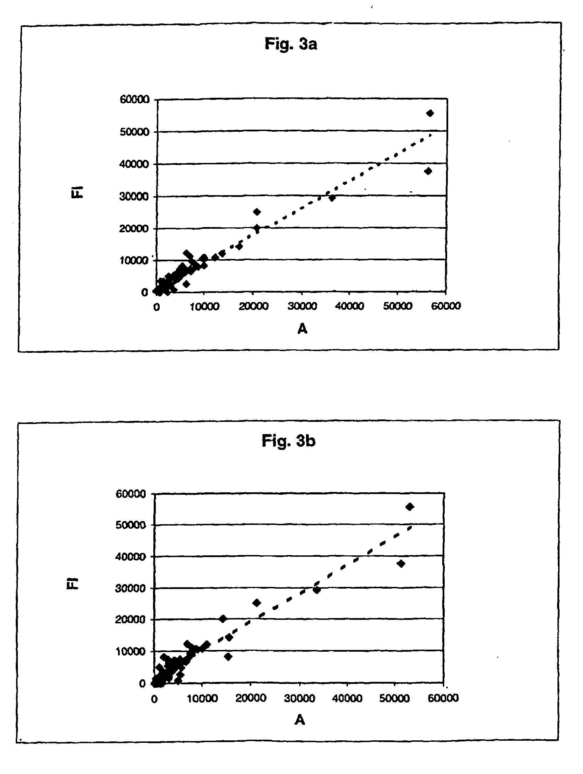

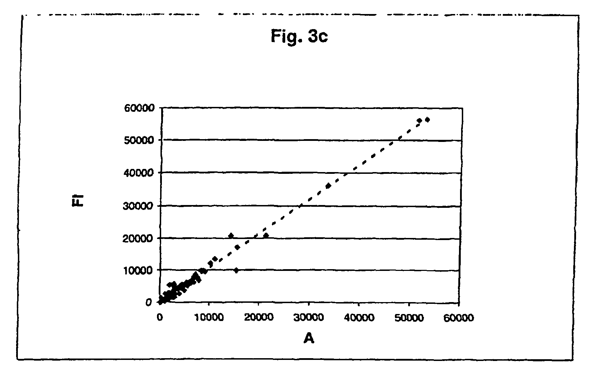

The mean standard deviation calculated from the values obtained after three individual assays was 12.36 %. This means that the chip-based immunological assay, questioning the presence of IgE in patients' sera, is highly reproducible. This is also evident when inspecting scatter-plots derived from the triple-assay 3, s. Fig. 3a-3c, wherein Fig. 3a shows a Scatterplot Test 1 vs. Test 3, Fig. 3b a Scatterplot Test 2 vs. Test 3 and Fig. 3c a Scatterplot Test 1 vs. Test 2; A showing the allergens and FI the fluorescence intensity.

### Example 4:

### Comparison of different microarrays

In order to test for the optimum slide for the present invention, individually prepared slides were evaluated according to a number of criterion that are outlined below:
- Production process: Slides that were prepared were evaluated according to a number of parameters, such as hazardous chemical requirement and production time.
- Cost of production: Slides produced in house and commercially available slides were compared according to their costs.
- Binding capacity: Different slides were evaluated according to the binding capacity of a fluorescent labelled protein.
- Reproducibility: Serial experiments were performed with differently pretreated slides and evaluated according to the overall assay performance.
- General Background: All slides were evaluated before and after an allergy assay for a systematic surface background that might diminish the signal to noise ratio.
- Detection limit: All slides were evaluated for the minimal protein concentration detectable per spot in an allergy screening assay.
- Serum tolerance: Generally, a patient's serum is a complex mixture of proteins that often interferes negatively with a microarray-based immunoassay with different batches of patients's serum.
- Blocking: All slides were evaluated for necessity of a blocking step prior to the allergy assay.
- Storage: All slides were evaluated for long-time storage after an allergen chip has been produced.

The results of the above mentioned evaluation study are depicted in table 2:

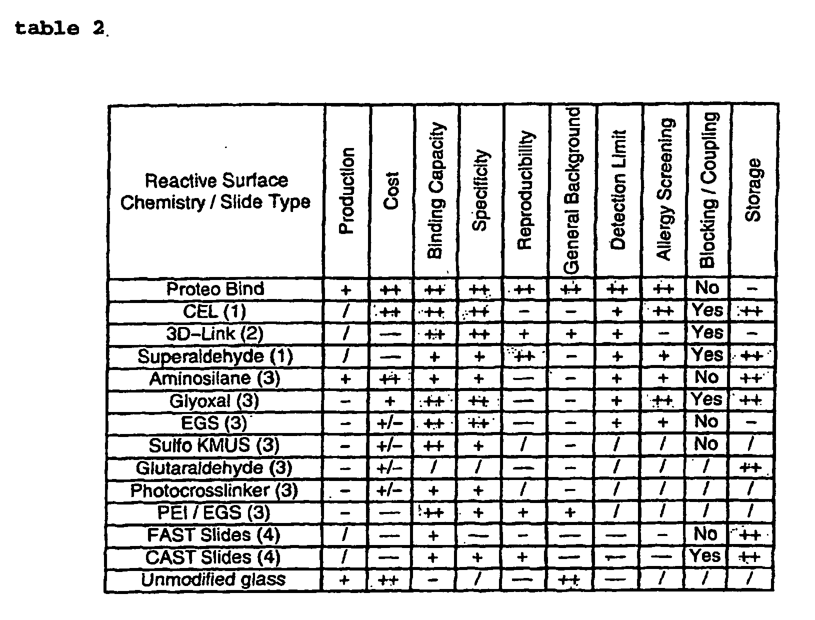

Table 2. Depicted are the results of the evaluation study described in the text. (++) means excellent result, (+) good, (-) minor, (--) bad. (/) means that the slide has not been assayed for this particular category. (1) Slides containing a functional surface with aldehyde groups. (2) Slides containing an amine-reactive surface, the exact chemical properties of which are not known. (3) Slides produced in-house with functional groups as mentioned in table 2. (4) Slides containing a membrane for immobilization of proteins. ProteoBind is the working title for the surface derivatisation adapted for an optimized performance of in microarray-based allergy diagnosis and comprises (1-[3"-[trimethoxysilyl)propyl]-1'(4"-isothiocyanatophenyl) thiourea), which was prepared according to Chen et al. (Nucleic Acids Research, 199, vol.27, No. 2), which is incorporated herein by reference.

According to the evaluation study presented above ProteoBind was selected as a superior surface derivatisation for the production of allergen microarrays.

### Example 5:

### Comparison of the detection of immunglobulin in a sample with immobilized recombinant allergens and with immobilized extract

In order to test the sensitivity and diagnostic relevant information of purified recombinant allergens immobilized to a microarray and of allergen extract immobilized to a microarray, specific allergens of one source were compared to an allergen extract of the same source.

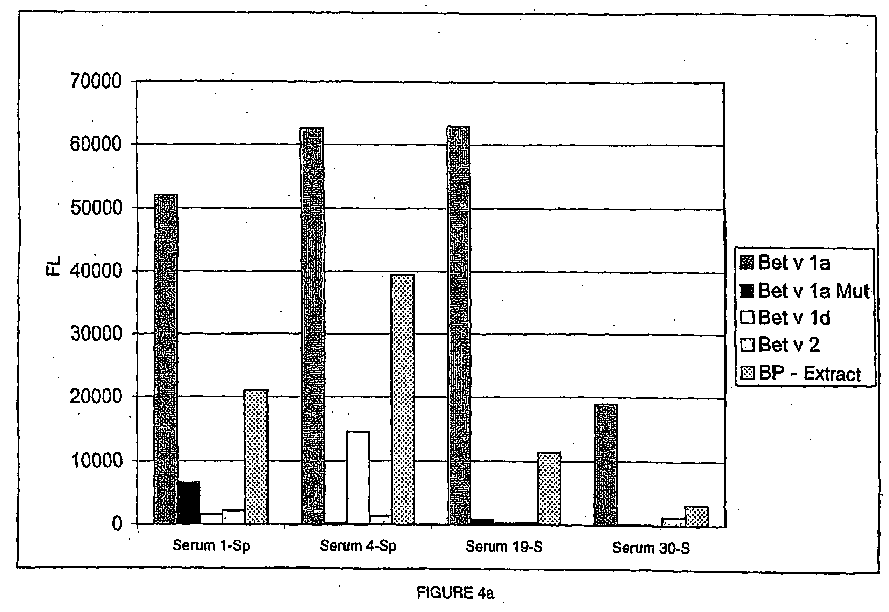

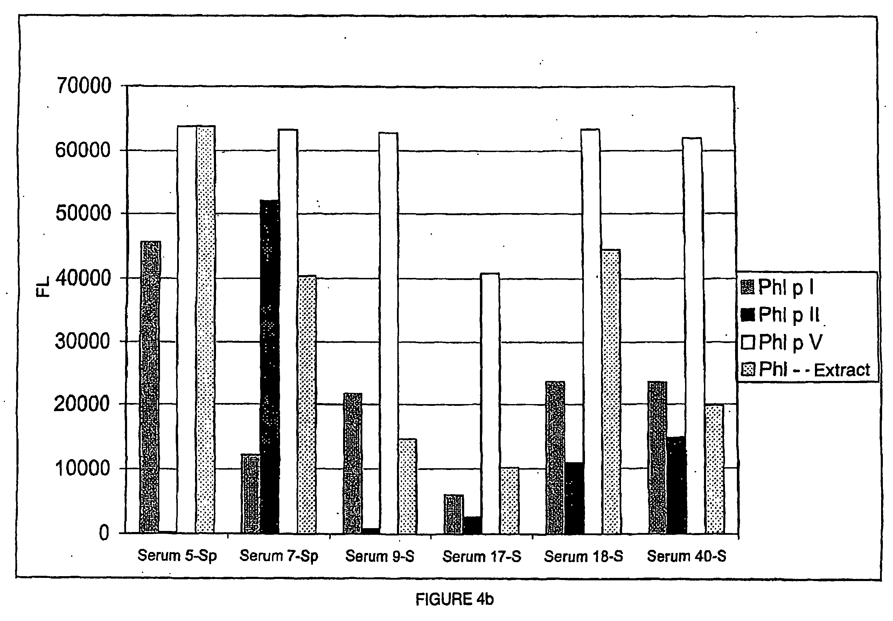

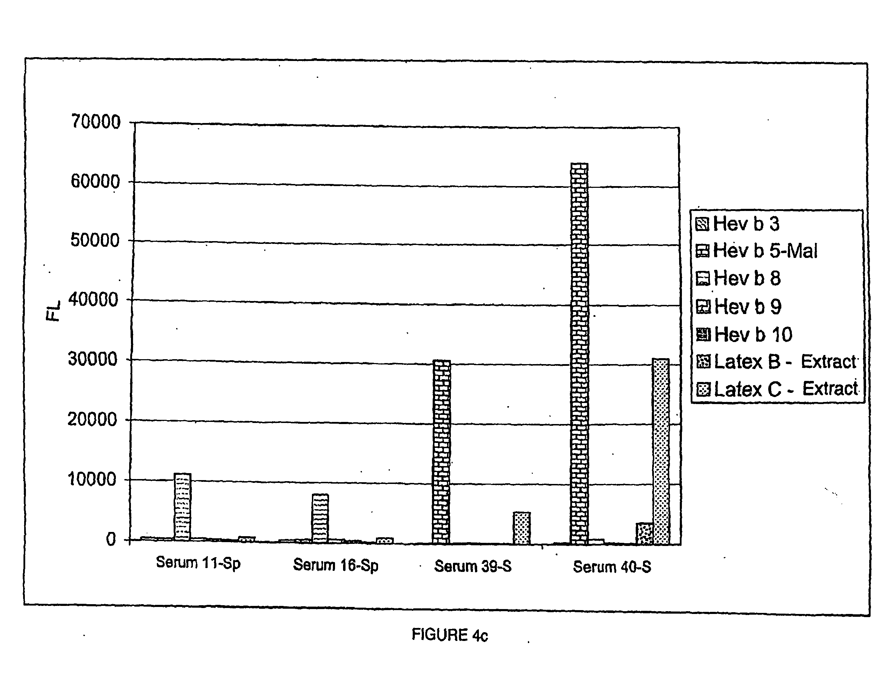

After immobilization of allergen compositions to a microarray different samples comprising said specific serum were added onto the mircroarray and the fluorescence which corresponds to the amount of antibody bound to the allergens was measured. The results are shown in tables 3, 4 and 5 as well as figures 4a, 4b and 4c, where "FL" stand for %fluorescence. From these results it is clear that in the case of the recombinant allergens, detection and quantification is much more sensitive than in the case of the allergen extracts. The fluorescence intensity of a single recombinant antigene is clearly higher than the fluorescence intensity of the extract. Furthermore, in the case of the extract only the source of the extract can be tested and it is not possible to test which specific antigene of the source is responsible for the allergy to the contrary of detection with recombinant allergens.

Therefore, the inventive method using single purified allergens, in particular recombinant allergens are advantageous over extracts comprising allergens.

**Table 3**

| Graph 1 | Serum 1-Sp | Serum 4-Sp | Serum 19-S | Serum 30-S |
|---|---|---|---|---|
| Bet v 1a | 52028 | 62529 | 62932 | 18847 |
| Bet v 1a Mut | 6590 | 237 | 905 | 139 |
| Bet v 1d | 1576 | 14352 | 252 | 89 |
| Bet v 2 | 2196 | 1408 | 273 | 1253 |
| BP - Extract | 21013 | 39438 | 11390 | 3174 |

**Table 4**

| Graph 2 | Serum 5-Sp | Serum 7-Sp | Serum 9-S | Serum 17-S | Serum 18-S | Serum 40-S |
|---|---|---|---|---|---|---|
| Phl p I | 45611 | 12148 | 21588 | 5838 | 23497 | 23424 |
| Phl p II | 209 | 52043 | 814 | 2562 | 10984 | 14753 |
| Phl p V | 64005 | 63481 | 62953 | 40770 | 63530 | 62029 |
| Phl - Extract | 63987 | 40318 | 14504 | 10183 | 44405 | 19798 |

**Table 5**

| Graph 3 | Serum 11-Sp | Serum 16-Sp | Serum 39-S | Serum 40-S |
|---|---|---|---|---|
| Hev b 3 | 416 | 387 | 108 | 235 |
| Hev b 5-Mal | 350 | 519 | 30601 | 63897 |
| Hev b 8 | 11146 | 7977 | 134 | 853 |
| Hev b 9 | 460 | 631 | 105 | 188 |
| Hev b 10 | 360 | 429 | 73 | 152 |
| Latex B - Extract | 264 | 202 | 80 | 3426 |
| Latex C - Extract | 769 | 866 | 5343 | 30970 |

## Claims

1. A method for the detection of an IgE immunoglobulin which binds to an allergen in a sample, **characterized in that** one or more purified single allergens are immobilized on a microarray chip after which the sample is incubated with the immobilized allergens so that IgE immunoglobulins which are specific for the allergens bind to the specific allergen after which the IgE immunoglobulins which are bound to the specific immobilized allergens are detected.

2. The method according to claim 1, **characterized in that** one or more recombinant allergens are immobilized on the microarray chip.

3. The method according to claims 1 or 2, **characterized in that** one or more synthetically produced allergens are immobilized on the microarray chip.

4. The method according to any one of claims 1 to 3, **characterized in that** one or more haptens are immobilized as allergens on the microarray chip.

5. The method according to any one of claims 1 to 4, **characterized in that** the allergens are immobilized on a 10 to 2000 *µ*m diameter spot on the microarray chip.

6. The method according to any one of claims 1 to 5, **characterized in that** the allergens are immobilized on a 50-500 *µ*m diameter, preferably a 150-250 *µ*m diameter, spot on the microarray chip.

7. The method according to any one of claims 1 to 6, **characterized in that** the allergens are immobilized on a solid support as the microarray chip.

8. The method according to any one of claims 1 to 6, **characterized in that** the allergens are immobilized on a glass and synthetic carrier, respectively, as the microarray chip.

9. The method according to any one of claims 1 to 6, **characterized in that** the allergens are immobilized on a silicon wafer as the microarray chip.

10. The method according to any one of claims 1 to 6, **characterized in that** the allergens are immobilized on a membrane as the microarray chip.

11. The method according to any one of claims 1 to 10, **characterized in that** the microarray chip is chemically modified, preferably by aminoreactive and carboxyreactive modification, respectively.

12. The method according to any one of claims 1 to 11, **characterized in that** the allergens are covalently bound to the microarray chip.

13. The method according to any one of claims 1 to 12, **characterized in that** the immunoglobulins are detected in blood serum as the sample.

14. The method according to claim 13, **characterized in that** the blood serum is diluted 1:1 - 1:15, preferably 1:5.

15. The method according to any one of claims 1 to 14, **characterized in that** the sample is incubated 1 min. to 24 hours, preferably 1 to 2 hours, with the allergens.

16. The method according to any one of claims 1 to 15, **characterized in that** the sample is incubated at a temperature between 0 and 60°C, preferably at 37°C, with the allergens.

17. The method according to any one of claims 1 to 16, **characterized in that** the bound immunoglobulins are detected with at least one labelled, specific anti-immunoglobulin antibody.

18. The method according to claim 17, **characterized in that** the bound immunoglobulins are detected with at least one fluorescence labelled specific anti-immunoglobulin antibody.

19. The method according to claim 17, **characterized in that** the bound immunoglobulins are detected with at least one radioactive labelled specific anti-immunoglobulin antibody.

20. The method according to any one of claims 1 to 19, **characterized in that** one or more indoor allergens are immobilized as allergens.

21. The method according to any one of claims 1 to 19, **characterized in that** one or more outdoor allergens are immobilized as allergens.

22. The method according to any one of claims 1 to 19, **characterized in that** one or more food allergens are immobilized as allergens.

23. The method according to any one of claims 1 to 19, **characterized in that** one or more venom allergens are immobilized as allergens.

24. A method for in vitro diagnosis of allergies in a patient, **characterized in that** a serum sample from the patient is analysed for IgE immunoglobulins which bind to allergens, according to a method according to any one of claims 1 to 23, whereby a microarray chip is used on which at least 10, preferably at least 50, still preferred at least 90, different allergens are immobilized, after which a positive reaction between the sample and the immobilized allergens is diagnosed as an allergy.

25. The use of a microarray chip on which one or more purified single allergens are immobilized for the detection of IgE immunoglobulins.

26. The use of a microarray chip according to claim 25 **characterized in that** the allergens are immobilized on a 100 to 500 *µ*m diameter, preferably 200-300 *µ*m diameter, spot.

27. The use of a microarray chip according to claim 25 or 26, **characterized in that** it is a glass carrier.

28. The use of a microarray chip according to claim 25 or 26, **characterized in that** it is a synthetic carrier.

29. The use of a microarray chip according to claim 25 or 26, **characterized in that** it is a silicon wafer.

30. The use of a microarray chip according to claim 25 or 26, **characterized in that** it is a membrane.

31. The use of a microarray chip according to any one of claims 25 to 30, **characterized in that** it is chemically modified, preferably by aminoreactive andcarboxyreactive modification, respectively.

32. The use of a microarray chip according to any one of claims 25 to 31, **characterized in that** the allergens are covalently bound to it.

33. The use of a kit for carrying out a method according to any one of claims 1 to 24, **characterized in that** it comprises a microarray chip on which one or more purified single allergens are immobilized and a first reagent comprising at least one immunoglobulin detecting reagent, preferably an anti-immunoglobulin antibody, preferably in a known concentration, and possibly a second reagent as a positive sample comprising at least one immunoglobulin which binds to an allergen.

## Patentansprüche

1. Verfahren zur Detektion eines IgE-Immunglobulins, das an ein Allergen in einer Probe bindet, **dadurch gekennzeichnet, dass** ein oder mehrere gereinigte einzelne Allergene auf einem Microarray-Chip immobilisiert werden, wonach anschließend die Probe mit den immobilisierten Allergenen inkubiert wird, so dass IgE-Immunglobuline, die für die Allergene spezifisch sind, an das spezifische Allergen binden, wonach die an die spezifischen immobilisierten Allergene gebundenen IgE-Immunglobuline detektiert werden.

2. Verfahren nach Anspruch 1, **dadurch gekennzeichnet, dass** ein oder mehrere rekombinante Allergene auf dem Microarray-Chip immobilisiert werden.

3. Verfahren nach Anspruch 1 oder 2, **dadurch gekennzeichnet, dass** ein oder mehrere synthetisch hergestellte Allergene auf dem Microarray-Chip immobilisiert werden.

4. Verfahren nach einem der Ansprüche 1 bis 3, **dadurch gekennzeichnet, dass** ein oder mehrere Haptene als Allergene auf dem Microarray-Chip immobilisiert werden.

5. Verfahren nach einem der Ansprüche 1 bis 4, **dadurch gekennzeichnet, dass** die Allergene auf einem Spot mit einem Durchmesser von 10 bis 2000 *µ*m auf dem Microarray-Chip immobilisiert werden.

6. Verfahren nach einem der Ansprüche 1 bis 5, **dadurch gekennzeichnet, dass** die Allergene auf einem Spot mit einem Durchmesser von 50 bis 500 *µ*m, vorzugsweise 150-250 *µ*m, auf dem Microarray-Chip immobilisiert werden.

7. Verfahren nach einem der Ansprüche 1 bis 6, **dadurch gekennzeichnet, dass** die Allergene auf einem festen Träger als Microarray-Chip immobilisiert werden.

8. Verfahren nach einem der Ansprüche 1 bis 6, **dadurch gekennzeichnet, dass** die Allergene auf einem Glas- bzw. Kunststoffträger als Microarray-Chip immobilisiert werden.

9. Verfahren nach einem der Ansprüche 1 bis 6, **dadurch gekennzeichnet, dass** die Allergene auf einem Siliziumplättchen als Microarray-Chip immobilisiert werden.

10. Verfahren nach einem der Ansprüche 1 bis 6, **dadurch gekennzeichnet, dass** die Allergene an einer Membran als Microarray-Chip immobilisiert werden.

11. Verfahren nach einem der Ansprüche 1 bis 10, **dadurch gekennzeichnet, dass** der Microarray-Chip chemisch modifiziert wird, vorzugsweise durch eine Amino-reaktive bzw. Carboxy-reaktive Modifizierung.

12. Verfahren nach einem der Ansprüche 1 bis 11, **dadurch gekennzeichnet, dass** die Allergene kovalent an den Microarray-Chip gebunden werden.

13. Verfahren nach einem der Ansprüche 1 bis 12, **dadurch gekennzeichnet, dass** die Immunglobuline in Blutserum als Probe detektiert werden.

14. Verfahren nach Anspruch 13, **dadurch gekennzeichnet, dass** das Blutserum 1:1 - 1:15, vorzugsweise 1:5 verdünnt wird.

15. Verfahren nach einem der Ansprüche 1 bis 14, **dadurch gekennzeichnet, dass** die Probe mit den Allergenen 1 min bis 24 Stunden, vorzugsweise 1 bis 2 Stunden, inkubiert wird.

16. Verfahren nach einem der Ansprüche 1 bis 15, **dadurch gekennzeichnet, dass** die Probe mit den Allergenen bei einer Temperatur zwischen 0 und 60°C, vorzugsweise bei 37°C inkubiert wird.

17. Verfahren nach einem der Ansprüche 1 bis 16, **dadurch gekennzeichnet, dass** die gebundenen Immunglobuline mit zumindest einem markierten, spezifischen anti-Immunglobulin-Antikörper detektiert werden.

18. Verfahren nach Anspruch 17, **dadurch gekennzeichnet, dass** die gebundenen Immunglobuline mit zumindest einem Fluoreszenz-markierten, spezifischen anti-Immunglobulin-Antikörper detektiert werden.

19. Verfahren nach Anspruch 17, **dadurch gekennzeichnet, dass** die gebundenen Immunglobuline mit zumindest einem radioaktiv markierten, spezifischen anti-Immunglobulin-Antikörper detektiert werden.

20. Verfahren nach einem der Ansprüche 1 bis 19, **dadurch gekennzeichnet, dass** ein oder mehrere Indoor-Allergene als Allergene immobilisiert werden.

21. Verfahren nach einem der Ansprüche 1 bis 19, **dadurch gekennzeichnet, dass** ein oder mehrere Outdoor-Allergene als Allergene immobilisiert werden.

22. Verfahren nach einem der Ansprüche 1 bis 19, **dadurch gekennzeichnet, dass** ein oder mehrere Nahrungsmittel-Allergene als Allergene immobilisiert werden.

23. Verfahren nach einem der Ansprüche 1 bis 19, **dadurch gekennzeichnet, dass** ein oder mehrere Gift-Allergene als Allergene immobilisiert werden.

24. Verfahren zur in vitro-Diagnose von Allergien in einem Patienten, **dadurch gekennzeichnet, dass** eine dem Patienten entnommene Serumprobe auf an Allergene bindende IgE-Immunglobuline gemäß einem Verfahren nach einem der Ansprüche 1 bis 23 analysiert wird, wobei ein Microarray-Chip verwendet wird, auf dem zumindest 10, vorzugsweise zumindest 50, mehr bevorzugt zumindest 90, verschiedene Allergene immobilisiert sind, wonach eine positive Reaktion zwischen der Probe und den immobilisierten Allergenen als Allergie diagnostiziert wird.

25. Verwendung eines Microarray-Chips, auf dem ein oder mehrere gereinigte einzelne Allergene immobilisiert sind, zur Detektion von IgE-Immunglobulinen.

26. Verwendung eines Microarray-Chips nach Anspruch 25, **dadurch gekennzeichnet, dass** die Allergene auf einem Spot mit einem Durchmesser von 100 bis 500 *µ*m, vorzugsweise 200-300 *µ*m, immobilisiert sind.

27. Verwendung eines Microarray-Chips nach Anspruch 25 oder 26, **dadurch gekennzeichnet, dass** er ein Glasträger ist.

28. Verwendung eines Microarray-Chips nach Anspruch 25 oder 26, **dadurch gekennzeichnet, dass** er ein Kunststoffträger ist.

29. Verwendung eines Microarray-Chips nach Anspruch 25 oder 26, **dadurch gekennzeichnet, dass** er ein Siliziumplättchen ist.

30. Verwendung eines Microarray-Chips nach Anspruch 25 oder 26, **dadurch gekennzeichnet, dass** er eine Membran ist.

31. Verwendung eines Microarray-Chips nach einem der Ansprüche 25 bis 30, **dadurch gekennzeichnet, dass** er chemisch modifiziert ist, vorzugsweise durch eine Amino-reaktive bzw. Carboxy-reaktive Modifizierung.

32. Verwendung eines Microarray-Chips nach einem der Ansprüche 25 bis 31, **dadurch gekennzeichnet, dass** die Allergene kovalent daran gebunden sind.

33. Verwendung eines Kits zur Durchführung des Verfahrens gemäß irgendeinem der Ansprüche 1 bis 24, **dadurch gekennzeichnet, dass** es einen Microarray-Chip, auf dem ein oder mehrere gereinigte einzelne Allergene immobilisiert sind, sowie ein erstes Reagens, umfassend zumindest ein Immunglobulin-detektierendes Reagens, vorzugsweise einen anti-Immunglobulin-Antikörper, vorzugsweise in einer bekannten Konzentration, sowie gegebenenfalls als positive Probe ein zweites Reagens, umfassend zumindest ein an ein Allergen bindendes Immunglobulin, umfasst.

## Revendications

1. Procédé de détection d'une immunoglobuline IgE qui se lie à un allergène dans un échantillon, **caractérisé en ce qu'**un ou plusieurs allergènes uniques purifiés sont immobilisés sur une puce puis l'échantillon est incubé avec les allergènes immobilisés de telle sorte que les immunoglobulines IgE qui sont spécifiques des allergènes se lient aux allergènes spécifiques puis les immunoglobulines IgE qui sont liées aux allergènes immobilisés spécifiques sont détectées.

2. Procédé selon la revendication 1, **caractérisé en ce qu'**un ou plusieurs allergènes recombinants sont immobilisés sur la puce.

3. Procédé selon la revendication 1 ou 2, **caractérisé en ce qu'**un ou plusieurs allergènes produits synthétiquement sont immobilisés sur la puce.

4. Procédé selon l'une quelconque des revendications 1 à 3, **caractérisé en ce qu'**un ou plusieurs haptènes sont immobilisés comme allergènes sur la puce.

5. Procédé selon l'une quelconque des revendications 1 à 4, **caractérisé en ce que** les allergènes sont immobilisés sur un point d'un diamètre de 10 à 2000 *µ*m sur la puce.

6. Procédé selon l'une quelconque des revendications 1 à 5, **caractérisé en ce que** les allergènes sont immobilisés sur un point d'un diamètre de 50-500 *µ*m, de préférence d'un diamètre de 150-250 *µ*m, sur la puce.

7. Procédé selon l'une quelconque des revendications 1 à 6, **caractérisé en ce que** les allergènes sont immobilisés sur un support solide en tant que puce.

8. Procédé selon l'une quelconque des revendications 1 à 6, **caractérisé en ce que** les allergènes sont immobilisés sur un support en verre et synthétique, respectivement, en tant que puce.

9. Procédé selon l'une quelconque des revendications 1 à 6, **caractérisé en ce que** les allergènes sont immobilisés sur une tranche de silicium en tant que puce.

10. Procédé selon l'une quelconque des revendications 1 à 6, **caractérisé en ce que** les allergènes sont immobilisés sur une membrane en tant que puce.

11. Procédé selon l'une quelconque des revendications 1 à 10, **caractérisé en ce que** la puce est chimiquement modifiée, de préférence par modification aminoréactive et carboxyréactive, respectivement.

12. Procédé selon l'une quelconque des revendications 1 à 11, **caractérisé en ce que** les allergènes sont liés de façon covalente à la puce.

13. Procédé selon l'une quelconque des revendications 1 à 12, **caractérisé en ce que** les immunoglobulines sont détectées dans du sérum sanguin comme l'échantillon.

14. Procédé selon la revendication 13, **caractérisé en ce que** le sérum sanguin est dilué 1:1 - 1:15, de préférence 1:5.

15. Procédé selon l'une quelconque des revendications 1 à 14, **caractérisé en ce que** l'échantillon est incubé 1 mn à 24 heures, de préférence 1 à 2 heures, avec les allergènes.

16. Procédé selon l'une quelconque des revendications 1 à 15, **caractérisé en ce que** l'échantillon est incubé à une température entre 0 et 60°C, de préférence à 37°C, avec les allergènes.

17. Procédé selon l'une quelconque des revendications 1 à 16, **caractérisé en ce que** les immunoglobulines liées sont détectées avec au moins un anticorps anti-immunoglobulines spécifique marqué.

18. Procédé selon la revendication 17, **caractérisé en ce que** les immunoglobulines liées sont détectées avec au moins un anticorps anti-immunoglobulines spécifique marqué par fluorescence.

19. Procédé selon la revendication 17, **caractérisé en ce que** les immunoglobulines liées sont détectées avec au moins un anticorps anti-immunoglobulines spécifique marqué par radioactivité.

20. Procédé selon l'une quelconque des revendications 1 à 19, **caractérisé en ce qu'**un ou plusieurs allergènes intérieurs sont immobilisés comme allergènes.

21. Procédé selon l'une quelconque des revendications 1 à 19, **caractérisé en ce qu'**un ou plusieurs allergènes extérieurs sont immobilisés comme allergènes.

22. Procédé selon l'une quelconque des revendications 1 à 19, **caractérisé en ce qu'**un ou plusieurs allergènes alimentaires sont immobilisés comme allergènes.

23. Procédé selon l'une quelconque des revendications 1 à 19, **caractérisé en ce qu'**un ou plusieurs allergènes de venin sont immobilisés comme allergènes.

24. Procédé de diagnostic in vitro d'allergies chez un patient, **caractérisé en ce qu'**un échantillon de sérum est prélevé au patient puis l'échantillon est analysé pour les immunoglobulines IgE qui se lient à des allergènes, selon un procédé selon l'une quelconque des revendications 1 à 23, par lequel une puce est utilisée sur laquelle au moins 10, de préférence au moins 50, plus préférentiellement au moins 90, allergènes différents sont immobilisés, puis une réaction positive entre l'échantillon et les allergènes immobilisés est diagnostiquée comme une allergie.

25. Utilisation d'une puce sur laquelle un ou plusieurs allergènes uniques purifiés sont immobilisés pour la détection d'immunoglobulines IgE.

26. Utilisation d'une puce selon la revendication 25, **caractérisée en ce que** les allergènes sont immobilisés sur un point d'un diamètre de 100 à 500 µm, de préférence d'un diamètre de 200 à 300 µm.

27. Utilisation d'une puce selon la revendication 25 ou 26, **caractérisée en ce qu'**elle est un support en verre.

28. Utilisation d'une puce selon la revendication 25 ou 26, **caractérisée en ce qu'**elle est un support synthétique.

29. Utilisation d'une puce selon la revendication 25 ou 26, **caractérisée en ce qu'**elle est une tranche de silicium.

30. Utilisation d'une puce selon la revendication 25 ou 26, **caractérisée en ce qu'**elle est une membrane.

31. Utilisation d'une puce selon l'une quelconque des revendications 25 à 30, **caractérisée en ce qu'**elle est chimiquement modifiée, de préférence par modification aminoréactive et carboxyréactive, respectivement.

32. Utilisation d'une puce selon l'une quelconque des revendications 25 à 31, **caractérisée en ce que** les allergènes sont liés à elle de façon covalente.

33. Utilisation d'un kit pour réaliser un procédé selon l'une quelconque des revendications 1 à 24, **caractérisé en ce qu'**il comprend une puce sur laquelle un ou plusieurs allergènes uniques purifiés sont immobilisés et un premier réactif comprenant au moins un réactif détectant des immunoglobulines, de préférence un anticorps anti-immunoglobulines, de préférence à une concentration connue, et éventuellement un second réactif comme échantillon positif comprenant au moins une immunoglobuline qui se lie à un allergène.
